(19) Europäisches Patentamt

European Patent Office

Office européen des brevets

(11) Publication number: **0 490 212 A1**

## EUROPEAN PATENT APPLICATION

(21) Application number: **91120658.9**

(22) Date of filing: **02.12.91**

(51) Int. Cl.5: **A61M 1/34**

(30) Priority: **07.12.90 IT 4016190**

(43) Date of publication of application:
**17.06.92 Bulletin 92/25**

(84) Designated Contracting States:
**AT BE CH DE DK ES FR GB GR IT LI LU NL SE**

(71) Applicant: **SIFRA S.p.A.**
**Via Camagre, 41-43**
**I-37063 Isola della Scala,(Verona)(IT)**

(72) Inventor: **La Mura, Umberto**
**Via Casarsa, 58**
**I-36100 Vicenza(IT)**

(74) Representative: **Modiano, Guido et al**
**MODIANO, JOSIF, PISANTY & STAUB**
**Modiano & Associati Via Meravigli, 16**
**I-20123 Milano(IT)**

(54) **High-efficiency apparatus for performing hemodialysis, hemofiltration plasmapheresis, and hemodiafiltration.**

(57) A high efficiency dialysis, hemofiltration plasmapheresis apparatus, artificially keeps blood flow through filter at optimal valve.

In case of insufficient blood supply from the patient, a recirculation pump-system activates a recirculation blood line that draws blood at the output tract of the blood filtration unit, and reinfuses it at the inflow tract of the blood filtration unit.

Polysaline solutions are added at the reinfusion site.

The apparatus includes a blood withdrawal line (2), a first pumping unit (4), a first drip feed unit (5), at the filtration unit input, the filtration unit itself (6), a second drip feed unit (10) at the filtration unit output, a recirculation line (12), a second pumping element (13), a station (14) for distributing polysaline solutions.

The present invention relates to a high-efficiency apparatus for performing hemodialysis, hemofiltration plasmapheresis, and hemodiafiltration.

In the field of dialysis, as well as of hemofiltration plasmapheresis and hemodiafiltration, there is an ongoing search for further improvements in the components used to perform these processes.

The known art has increasingly refined its ability to produce, for example, filters with progressively more efficient membranes or increasingly effective blood "cleaning" solutions.

In this field, however, it has been observed that despite the various improvements achieved mainly in filter structures, said filters can operate with efficiencies which are advantageous for the patients when the blood flow which transits through them approaches, or reaches, the optimum value of 300 ml/minute.

This flow-rate is objectively rather high, and not all dialysed patients can release, in the unit time, the above mentioned amount (flow-rate) of blood so that it can be introduced in the circuit and through the filter, where the contained toxic substances are physically removed.

The low flow-rates at which the apparatus is sometimes forced to work (when, for example, the patient is a senior person, a person affected by vascular problems, or a multiple-trauma patient, for whom the use of a peripheral and thus scarcely supplied withdrawal point is necessary), cause the exchange between blood and cleaning liquid inside the filter to be proportionally reduced in percentage, since said exchange is normally a function of a series of parameters such as for example the thickness and type of membrane used in the filter, the relative thicknesses of the hematic film and of the dialysis liquid, the hematic flow and the flow of the dialysis liquid, and others.

Thus, the efficiency of a dialysis apparatus is already unfortunately substantially limited in "normal" conditions; this is even more true in the extreme cases exemplified above.

Furthermore, by using pathways for so-called access to the patient which notoriously have a considerable blood flow-rate, such as the femoral artery or the jugular vein or the subclavian vessels, the risk of infections due to the insertion of the catheters and to their subsequent handling may arise.

The technical aim of the present invention is to solve the problems of the known art by providing a high-efficiency apparatus for performing hemodialysis, hemofiltration plasmapheresis, and hemodiafiltration, which allows to use the filtration assembly in optimum operating and efficiency conditions even with limited hematic flow-rates, which furthermore allows to use scarcely supplied accesses to the patient's circulatory system, and which furthermore allows to introduce solutions with high purifying ability or, for equal normal conditions of the patient, to provide a significant overall reduction in said patient's therapy times.

This aim and these objects are achieved by a high-efficiency apparatus for performing hemodialysis, hemofiltration plasmapheresis, and hemodiafiltration, according to the present invention, as defined in the appended claims.

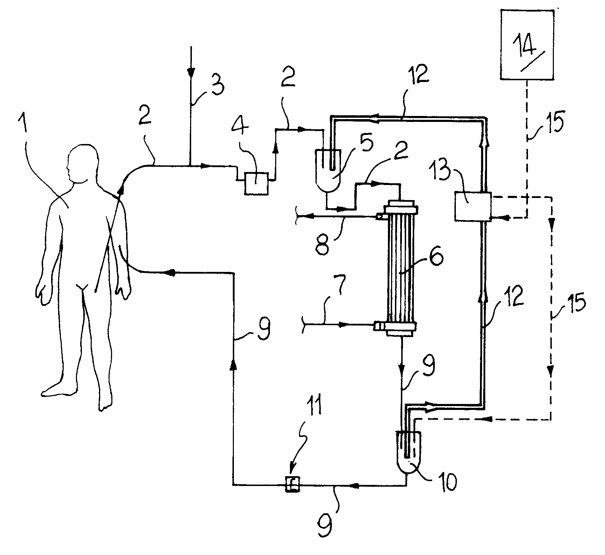

Further characteristics and advantages of the invention will become apparent from the description of a preferred but not exclusive embodiment of a high-efficiency apparatus for performing hemodialysis, hemofiltration plasmapheresis, and hemodiafiltration, according to the present invention, illustrated only by way of non-limitative example in the accompanying drawing, wherein the only figure is a schematic view of a high-efficiency apparatus according to the present invention.

With particular reference to the above figure, the reference numeral 1 designates a patient from whom there extends an arterial line 2 into which an additional anticoagulating drug feed line 3 leads.

The movement of the blood in the line 2 is activated by a peristaltic pumping unit 4 which pushes said blood into a first arterial drip-feed unit 5.

At the outlet of said arterial drip-feed unit, the line 2 leads into a filtration element 6 which is also entered by a duct 7 for the countercurrent feeding of a conventional dialytic purifying physiological solution which can be discharged by means of a further related duct 8.

At the outlet of the filtration element 6, a venous line 9 connects said filtration element 6 to a second venous drip-feed unit 10 which in turn returns to the patient 1 with the continuation of the venous line 9, after the interposition of a one-way valve means 11.

A second line 12 is provided in parallel to the line which mutually interconnects the first drip-feed unit 5, the filtration element 6 and the second venous drip-feed unit 10; said second line draws from said second drip-feed unit, is activated by a related second peristaltic pumping unit 13, and draws presettable volumes of blood, reintroducing them into the first arterial drip-feed unit 5 during treatment.

Optionally, there is a station 14 for introducing polysaline solutions into the second venous drip-feed unit 10 by means of a line 15 of its own.

The operation of the invention can be intuitively deduced from the above description: the conventional flow of blood leaving the patient 1 is pushed by the first pumping unit 4, after the introduction of anticoagulating drugs, through the additional line 3 into the arterial line 2 and into the first arterial drip-feed unit 5; the outlet of the second parallel line 12 also exits into said arterial drip-feed unit 5 and introduces therein preset volumes of blood drawn, with the aid of a second pumping unit 13, from the second drip-feed unit 10; by adding themselves to the blood drawn from the patient 1, said "additional" volumes ensure, upstream of the filtration element 6, a blood flow-rate which is constant and substantially coincides with the values for optimization of the operating efficiency of said element.

In addition to this, by introducing polysaline solutions arriving from the station 14 into the second venous drip-feed unit 10, said solutions are carried upstream of the filtration element 6 and, by being fed into it, contribute with a convective entrainment component to increase the percentage of elimination of so-called catabolites which have low/medium relative molecular mass, such as for example urea, creatinine, uric acid, various phosphates and others.

In practice it has been observed that the invention thus described achieves the intended aim and objects, i.e. it is able to compensate for scarce blood flow-rates due to senior or multiple-trauma patients or it furthermore allows to use a low-supply point as "source" from the patient without thus causing traumas at the larger vascular access points due to the various operating manoeuvres to be performed during treatment.

The invention thus conceived is susceptible to numerous modifications and variations, all of which are within the scope of the inventive concept.

All the details may furthermore be replaced with other technically equivalent elements.

In practice, the materials employed, as well as the dimensions, may be any according to the requirements without thereby abandoning the scope of the protection of the following claims.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. High-efficiency apparatus for performing hemodialysis, hemofiltration plasmapheresis, and hemodiafiltration, comprising an arterial line (2) for the outflow of blood to be purified from a patient, said arterial line being activated by means of a pumping unit and leading into a first arterial drip-feed unit (5) arranged upstream of an inlet of a purifying filtration element (6) whose output is connected to a second venous drip-feed unit (10) which is in turn connected, by means of a venous return line (9) on which a one-way valve means (11) is interposed, back to said patient, characterized in that at least one second line (12) is provided in parallel to said line connecting the first arterial drip-feed unit (5), the purifying filtration element (6) and the second venous drip-feed unit (10), said second line (12) being activated by means of a second pumping unit (13) for withdrawing presettable volumes of blood from the second venous drip-feed unit (10) and for reintroducing said volumes into said first drip-feed unit (10).

2. High-efficiency apparatus according to claim 1, characterized in that the overall hematic flow-rates entering said first arterial drip-feed unit (5) and said filtration element (6) have a constant value comprised between 250 and 350 ml/minute.

3. High-efficiency apparatus according to claim 1, characterized in that a station (14) for introducing polysaline solutions into the second venous drip-feed unit (10) is furthermore provided.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-8 102 979 (BAXTER)<br>* page 11, line 29 - page 12, line 24; figure 2 *<br>--- | 1,3 | A61M1/34 |
| A | US-A-4 411 792 (BABB)<br>* column 4, line 58 - line 60; figure 1 *<br>--- | 1 | |
| X | WO-A-7 901 121 (U.S.GOVERNMENT)<br>* page 12, line 23 - page 13, line 17; figure 1 *<br><br>* page 19, line 19 - line 34; figure 2 *<br><br>----- | 1,2 | |

TECHNICAL FIELDS SEARCHED (Int. Cl.5 )

A61M

The present search report has been drawn up for all claims

| Place of search | Date of completion of the search | Examiner |
|---|---|---|
| THE HAGUE | 19 FEBRUARY 1992 | PAPONE F. |